# EUROPEAN PATENT APPLICATION

(11) **EP 2 245 921 A1**
(43) Date of publication of application: **03.11.2010**
(21) Application number: 09703516.6
(22) Date of filing: 21.01.2009
(51) Int. Cl.: A01H 5/00, A23L 1/22, A23L 1/226, C12P 19/56, C12Q 1/68, C12N 15/09

(54) **NOVEL STEVIA VARIETY AND METHOD OF PRODUCING SWEETENER**

(30) Priority: 22.01.2008 JP 2008011756
(71) Applicant: Morita Kagaku Kogyo Co., Ltd., Osaka-shi, Osaka 536-0003 (JP)
(72) Inventor: MORITA, Toyoshige, Osaka-shi Osaka 536-0003 (JP); MORITA, Koji, Osaka-shi Osaka 536-0003 (JP); KANZAKI, Shinya, Souraku-gun Kyoto 619-0240 (JP)
(74) Representative: Atkinson, Jennifer
(86) International application number: PCT/JP2009/050878
(87) International publication number: WO 2009/093610

(57) **Abstract**

High-purity stevioside sweeteners are being provided. Stevia Rebaudiana Bertoni varieties were identified using DNA analysis after cross and selective breeding were implemented. By crossing these varieties, a novel variety which enables continuous cultivation of the specific variety was developed (Deposition No. FERM BP-10870). Extracting the dried leaves of this variety assures consistent high-concentration of stevioside, which makes it possible to produce various favorable stevioside sweeteners.

SEQUENCE LISTING
<110>  Morita Kagaku Kogyo Co.,Ltd.

<120>  Novel Stevia Variety and Method of Producing Sweetener

<130>  JA55670P.EPP

<150>  JP 2008-011756
<151>  2008-01-22

<150>  PCT/JP2009/050878
<151>  2009-01-21

<160>  2

<210>  1
<211>  23
<212>  DNA
<213>  Artificial Sequence

<220>
<223>  Characteristic DNA sequence found in improved species of stevia

<400>  1

cacgcgaact cctcgactcg acc                                              23


<210>  2
<211>  25
<212>  DNA
<213>  Artificial Sequence

<220>
<223>  Characteristic DNA sequence found in improved species of stevia

<400>  2
gctgcatgct tgcatgcatg aaatc                                            25

## Description

### TECHNICAL FIELD

The present invention relates to a plant belonging to the variety of Stevia Rebaudiana Bertoni which has an extremely-high content rate of stevioside and can be continuously produced from a seed, all plants which can be produced by using a seed obtained from the plant in question, and/or a method for preparing a sweetener which is extracted from dried leaves thereof, and a method for preparing α-glucosylstevioside from the sweetener.

### BACKGROUND ART

Stevia is an asteraceae perennial plant which is originated in Paraguay in South America with a scientific name: Stevia Rebaudiana Bertoni. Stevia is cultivated for extracting the sweetening component to use as a natural sweetener because it contains components which are more than 300 times sweeter than sugar.
Stevioside (C₃₈H₆₀O₁₈), rebaudioside A (C₄₄H₇₀O₂₃), rebaudioside C, D, E, dulcoside A and the like are known as the sweetening components of stevia. The varieties of stevia which are widely cultivated contain stevioside (hereinafter referred to as ST) as a major component of the above sweetening components. The content of rebaudioside A (hereinafter referred to as RA) is around 40 parts by weight against 100 parts by weight of ST and rebaudioside C is contained something less than RA. However, major components vary among different varieties. For example, there are varieties which contain rebaudioside C as their major component.

ST is widely used as a natural sweetener in the food industry because it has a degree of sweetness more than 300 times higher than a sugar. Although sweetness of ST is relatively similar to that of sugar, it is known that an unpleasant taste such as bitterness is left as an aftertaste compared to RA. In contrast, because RA has good quality sweetness and 1.3 to 1.5 times higher degree of sweetness than ST, stevia sweeteners which contain RA rather than ST as a major component are generally preferred.
For example, the present inventors have made a variety improvement by repeated crossing and selection of the original variety and obtained stevia varieties containing a very small quantity of ST compared to RA, from the seeds of these varieties seeding can be easily reared and the sweetening components can be continuously cultivated (see the patent documents 1 and 2 below).
However, the quality of sweetness is particularly subtle among tastes such as astringency and pungency, which are perceived by the tongue. Even ST which compared to RA is believed to have an unpleasant bitter aftertaste, if purified and its concentration is increased, off-tastes are being removed and its sweetness improves and thus ST can be used in a broader range of applications. For example, the sweetness of ST felt in the aftertaste can be used for masking the salty astringency in salty foods. In addition, ST can be used as a masking agent for the astringency, the organic acid and the salts of fruit juices and therefore, there is a demand for ST sweeteners as a different sweetener from RA in the food product field.
There is a method for improving the sweetness of a stevia extract by α-glucosylation and subsequent sugar chain adjustment (see the patent document 3 below). Typical of a sweetener containing α-glucosylstevioside as a main component prepared by the above method, if compared to a common stevia extract, is the less interference the sweet components other than ST have on sweetness quality.
It is also preferable in terms of effective utilization of resources to promote the use of ST because it is a main component of stevia sweeteners.
[Patent document 1] JP-A-2002-262822
[Patent document 2] WO2006/093229
[Patent document 3] JP-A-H02-163056

### DISCLOSURE OF INVENTION

### PROBLEMS TO BE SOLVED BY THE INVENTION

In the past, the only method for obtaining a high-purity ST was by repeated recrystallizations. Any components other than ST in stevia result in a less yield of recrystallization of ST. Thus, it was necessary to develop a novel stevia variety which has a lower RA content, which is the next main component in stevia, in order to reduce the production cost of high-purity ST and keep its stable yield.
In addition, it has been considered impossible to keep the sweetening components constant in seed cultivation as in stevia plant breeding it is impossible to identify the variety only by the height of the plant or the shape of its leafs, and also, the stevia plant is easily hybridized due to self-incompatibility.

### MEANS FOR SOLVING PROBLEMS

The present inventors made a variety improvement by repeated crossing and selection of the original variety and obtained a stevia variety having a significantly smaller quantity of RA than ST. They succeeded in development of a novel stevia variety from a seed of the above stevia variety wherein a seeding can be easily reared and the sweetening components can be continuously cultivated.

### EFFECT OF INVENTION

An extract obtained from the dried leaves of the present invention's novel stevia variety has a low RA content and a high ST content, and therefore, a ST sweetener which virtually contains no RA can be obtained with one recrystallization. Alternatively, ST sweetener with improved quality of sweetness can be obtained by treating the extract with enzymes.

### BEST MODE FOR CARRYING OUT THE INVENTION

The first embodiment of the present invention is a Stevia Rebaudiana Bertoni variety which contains not more than 8 parts by weight of RA against 100 parts by weight of ST. For this purpose, a novel plant belonging to the Stevia Rebaudiana Bertoni variety, which contains not more than 8 parts by weight of RA against 100 parts by weight of ST, was created by repeated crossing and selection as described hereinafter and identified the variety by a DNA analysis using a particular primer.

The second embodiment of the present invention is a method for preparing a high-purity ST sweetener which contains not more than 8 parts by weight of RA against 100 parts by weight of ST, wherein the above plant or its dried leaves are extracted with water or aqueous solvent, and then, the obtained crude product is recrystallized.

The third embodiment of the present invention is a method for preparing an α-glucosylstevioside sweetener wherein the above plant or its dried leaves are extracted with water or aqueous solvent, and then, the obtained crude product or a high-purity ST obtained by recrystallization of the crude product are reacted with, for example, cyclodextrin glucosyltransferase in order to add a glucose by α-addition.

The fourth embodiment of the present invention is a method for preparing an α-glucosylated stevioside sweetener in which a sugar chain adjustment is made, wherein the sweetener obtained by the above third embodiment is treated by α-1,4-glucosidase such as glucoamylase to adjust the added sugar chain.

The fifth embodiment of the present invention is a DNA analysis by a RAPD (Random Amplified Polymorphic DNA) method using a particular primer as described hereinafter.

For achieving these objects, the present inventors carried out variety improvement by repeated crossing and selection. In this case, a method for identifying the selected variety is important. As described above, stevia has strong self-incompatibility, so it is important to make crossing between identified varieties. For this purpose the present inventors examined a method for identifying a variety by DNA analysis.
The RAPD method is one of DNA analysis techniques and is a method which is comprised of conducting PCR (polymerase chain reaction) using multiple primers to amplify a DNA area which is caught between the sequences which are the same as or similar to those of the primers used, and analyzing a pattern of the amplified DNA by electrophoresis. In addition, cetyl trimethyl ammonium bromide (CTAB) is a quaternary ammonium base having long-chain alkyl group and can be used for isolation of a nucleic acid because it forms an insoluble complex with a polyanion such as a nucleic acid.

The method for identifying a variety on the basis of the difference in DNA is comprised of isolating a genomic DNA from a plant by CTAB, removing a ribonuculeic acid (RNA), obtaining a PCR amplification product by a PCR method using a primer mixture for subjecting to electrophoresis and distinguish the PCR amplification product on the basis of the difference between the obtained fingerprints. For a plant of the present invention, it was confirmed that there was a characteristic base sequence located upstream of the 210 bp as described below.
In practice, selectively precipitated genomic DNA is taken as a template of the raw material plant and the following sequences are being used as primers:
Forward primer: CACGCGAACTCCTCGACTCGACC
Reverse primer: GCTGCATGCTTGCATGCATGAAATC
   The following PCR cycles were used: 35 cycles at 96 °C for 10 sec, at 65°C for 30 min, at 72°C for 30 sec and further at 72 °C for 2 min. After the reaction, the PCR product was fractionated by 6 % modified polyacrylamide electrophoresis, stained by SYBR Gold and visualized by a blue light transilluminator. As a result, a band indicating polymorphism was detected at around 190 bp and 210 bp and a genotype of F1 type was confirmed in the tested 10 varieties. It was confirmed that these bands were amplified with good reproductivity, and therefore, a plant can be identified by a particular DNA band.

A method for preparing a sweetener is comprised of extracting the plant or dried leaves thereof with water and/or aqueous organic solvent, and then, concentrating the obtained extract; or is comprised of extracting as described above, optionally purifying the extract by removing ionic impurities using, for example, cation-exchange resin, anion-exchange resin or activated charcoal, adsorbing the sweetening components with adsorption resin, eluting it with hydrophilic solvent, optionally concentrating the eluant by treating with, for example, cation-exchange resin, anion-exchange resin or activated charcoal and drying it. The sweetener preparation method may include conventional purification means such as decolorization and the method for preparing of high-purity ST may include conventional means such as membrane separation, alcohol extraction or crystallization. In addition, a method of crystallization may use an organic solvent such as ethanol or methanol to which optionally water may be added. Other natural or artificial sweeteners, diluents etc. may be added to the obtained sweetener.

The obtained sweetener can be used for calorie reduction, sugar reduction, lowering of melting point, sweetness quality improvement and masking in candies, jellies, beverages, beverage powders, instant noodles, jams, frozen desserts, chewing gums, Japanese confectioneries, healthy foods, chocolates, tabletop sweeteners, baked confectioneries, delicacies boiled seafood-paste products, lactic acid beverages, lactobacillus beverages, coffee beverages, cocoa beverages, tea beverages, liqueurs, wines, sherbets, cereals, plant-fiber containing foods, sauces, soy sauces, bean pastes, vinegars dressings, mayonnaises, ketchups, curries, soups, rice confectioneries, cubic rice crackers, breads, biscuits, crackers, pancake mixes, canned fruits, canned vegetables, meat products, boiled fish-paste products, salty foods, pickles, seasoning mixes, luxury foods, cosmetics and the like. Natural high-intensity sweeteners such as licorice glycyrrhizin, luohanguo (monk fruit) and thaumatin; artificial sweeteners such as sodium cyclamate, saccharine sodium, aspartame, sucralose and neotame; and diluents such as oligosaccharide, sugar alcohol, sugar and dextrin may be further added.

### Breeding process of variety which contains ST at high concentration

Breeding was performed by crossing and selection of the original stevia variety. First, SS varieties containing higher concentration of ST compared to the original variety were crossed and the SS-1 varieties containing relatively high concentration of ST were selected from the obtained seeds. The selected varieties were further subjected to artificial crossing and the varieties containing lower concentration of RA were selected from the obtained seeds. Then, 10 varieties containing less than 5 parts by weight of RA against 100 parts by weight of ST were developed by further crossing among the selected varieties, and then, 9 varieties named F1ST-1 to F1ST-9 were identified as a high ST variety after excluding one variety due to a lack of cold hardiness.
The seeds obtained from these high ST varieties were seeded to cultivate a plant and analyzed dried leaves obtained from the plant to confirm that the sweetening component thereof did not differ from that of high ST varieties. Then, the genes of the varieties were sequenced and varieties sharing the same genes were identified as a F1ST variety.

In addition, the International Deposit of the seeds obtained from the F1ST varieties of the present invention was completed by the applicant (International Patent Organism Depository of National Institute of Advanced Industrial Science and Technology: Central 6, 1-1-1 Higashi, Tsukuba, Ibaraki, Japan; July 13, 2007; Accession Number FERM BP-10870). Accordingly, a plant of the present invention is easily available from the deposited seeds of the F1ST variety. Stevia is a self-incompatibility plant and it was thought that the seeds thereof can not always provide the desired plant. However, the crossing using the variety obtained by selection according to the DNA analysis described herein can always provide the desired plant.
If necessary, the variety of the present invention may be crossed with another stevia variety. Then, a plant containing a high concentration of ST would be very easily available by the selection as described in Example 1 below. All of these plants are included within the plants which can be obtained from the deposited seeds of the ST variety.

### EXAMPLES

The breeding process and its characteristics are illustrated in more details below. The present invention is not limited to the following breeding process or cultural systems.

### Example 1

In 2001, varieties containing relatively high concentration of ST were selected from the seeds obtained by crossing of the varieties containing relatively high concentration of ST and were crossed artificially in a plastic greenhouse in Niimi factory of Morita Kagaku Kogyo Co., Ltd. In March 2002, the obtained seeds were seeded in a plastic greenhouse in the Niimi factory and the germinated seedings were transplanted into a pot for raising seedings. In early May, 500 seedings having a height of more than around 8 cm were transplanted into a breeding field in the factory after two weeks from fertilization with 20 kg/10 are of nitrogen, phosphorous and potassium fertilizer components each. In early July, 10 kg/ 10 are of nitrogen, phosphorous and potassium fertilizer components each were fertilized as additional fertilization.

In early September, the strain of SS varieties having less than 30 parts by weight of RA against 100 parts by weight of ST was selected by herborizing and analyzing their sweetening components. In 2002, the SS varieties were crossed artificially in a plastic greenhouse in the Niimi factory. In March 2003, the obtained seeds were seeded in a plastic greenhouse in the Niimi factory and the germinated seedings were transplanted into pots for raising seedings. In early May, 300 seedings having a height of around 7 cm were transplanted into a breeding field in the factory after two weeks from fertilization with 20 kg/10 are of nitrogen, phosphorous and potassium fertilizer components each. In early July, 10 kg/10 are of nitrogen, phosphorous and potassium fertilizer components each were fertilized as additional fertilization.

In early September, the strain of SS-1 varieties having less than 15 parts by weight of RA against 100 parts by weight of ST was selected by herborizing and analyzing their sweetening components. In 2003, the strains were crossed. In March 2004, the obtained seeds were seeded in a plastic greenhouse in the Niimi factory and the germinated seedings were transplanted into pots for raising seedings. In early May, 300 seedings having a height of around 7 cm were transplanted into a breeding field in the factory after two weeks from fertilization with 20 kg/10 are of nitrogen, phosphorous and potassium fertilizer components each. In early July, 10 kg/10 are of nitrogen, phosphorous and potassium fertilizer components each were fertilized as additional fertilization.

In early September, the strain having less than 8 parts by weight of RA against 100 parts by weight of ST was identified as a F1ST variety by herborizing and analyzing their sweetening components. In 2004, the F1ST varieties were crossed. In March 2005, the obtained seeds were seeded in a plastic greenhouse in the Niimi factory and the germinated seedings were transplanted into a pot for raising seedings. In early May, 1,000 seedings having a height of around 7 cm were transplanted into a breeding field in the factory after two weeks from fertilization with 20 kg/10 are of nitrogen, phosphorous and potassium fertilizer components each. In early July, 10 kg/10 are of nitrogen, phosphorous and potassium fertilizer components each were fertilized as additional fertilization.
In early September, it was confirmed that there is less than 8 parts by weight of RA against 100 parts by weight of ST in the dried leaves after harvesting all plants and analyzing the sweetening components thereof.

### Comparative experiment 1 (May)

In order to compare a ST variety and another variety SN, in March 2006, 1,500 seeds obtained from the F1ST variety and the SN variety each were seeded in a plastic greenhouse in the Niimi factory and the germinated seedings were transplanted into pots for raising seedings. In early May, 400 seedings of each variety were grown to the same height as previously described and were transplanted into a breeding field in the factory after two weeks from fertilization with 20 kg/10 are of nitrogen, phosphorous and potassium fertilizer components each.

In mid-May, leaves were separated from 200 rods of the ST variety and the SN variety each and dried for analysis samples. The sweetening components were measured by a high-performance liquid chromatography under the following conditions.

### <High-performance chromatography>

Column: LiChrosolv NH₂, 5 µ, 4 mm x 250 mm
Flow Rate: 1.5 ml/min
Developing Solvent: acetonitrile:water = 82: 8
Wavelength: 210 nm
The following results were obtained:

**[Table 1]**

| Variety Name | ST (%) | RA (%) | RA ratio against 100 parts of ST |
|---|---|---|---|
| F1ST-1 | 6.9 | 0.2 | 2 |
| F1ST-2 | 6.5 | 0 | 0 |
| F1ST-3 | 6.8 | 0 | 0 |
| F1ST-4 | 6.2 | 0.1 | 1 |
| F1ST-5 | 7.0 | 0 | 0 |
| F1ST-6 | 6.0 | 0.2 | 3 |
| F1ST-7 | 5.9 | 0 | 0 |
| F1ST-8 | 6.7 | 0.3 | 4 |
| F1ST-9 | 8.2 | 0.5 | 6 |
| SN | 7.2 | 2.8 | 38.8 |

### Comparative experiment 2 (September)

In mid-July, an additional fertilization was done. In early September, 150 rods of each variety were cut just above the ground and leaves were separated. The leaves were dried and the sweetening components were measured for using as analysis samples.
The following results were obtained:

**[Table 2]**

| Variety Name | ST (%) | RA (%) | RA ratio against 100 parts of ST |
|---|---|---|---|
| F1ST-1 | 12.12 | 0.6 | 5 |
| F1ST-2 | 11.22 | 0.0 | 0 |
| F1ST-3 | 12.10 | 0.0 | 0 |
| F1ST-4 | 10.56 | 0.2 | 1 |
| F1ST-5 | 13.05 | 0.0 | 0 |
| F1ST-6 | 10.84 | 0.6 | 5 |
| F1ST-7 | 9.80 | 0.0 | 0 |
| F1ST-8 | 12.50 | 1.0 | 8 |
| F1ST-9 | 14.20 | 0.8 | 5 |
| SN | 9.8 | 3.9 | 39 |

In the F1ST variety and the SN variety, a tendency of increase in the content of the sweetening components as the plants grow up was observed. However, a large increase of the RA content was not observed in the F1ST variety.

### Identification of base sequences of genes

### <DNA extraction>

DNA extraction was performed by the CTAB method. About 0.2 g of the leaves collected from each sample is frozen in mortar with liquid nitrogen and pulverized with pestle. The pulverized sample was mixed in 1.5 ml micro-tube with 0.5 ml of 2 % CTBA solution (100mM Tris-HCl pH 8.0, 20mM EDTA pH 8.0, 2% CTAB, 1.4M NaCl, 1% PVP) and incubated for 30 minutes at 65 °C. 0.5 ml of chloroform/isoamyl alcohol (24:1) is added and stirred for 10 minutes, followed by centrifugation for 15 minutes at 1,500 rpm, and then, the aqueous layer was transferred to another 1.5 ml micro-tube. An equal amount of 100 % isopropanol was added and centrifuged for 15 minutes at 1,500 rpm, followed by rinse with 75 % ethanol, and then, dried and dissolved in 400 µl of a TE buffer. 1 µl of an RNase solution was added and incubated for 1 hour at 37 °C, and then, an equal amount of TE-saturated phenol was added, followed by centrifugation for 15 minutes at 1,500 rpm. The aqueous layer was transferred to another micro-tube and 1/10 amount of 3 M sodium acetate and an equal amount of isopropanol were added and mixed, followed by centrifugation for 30 minutes at 1,500 rpm. The resultant precipitation was rinsed twice with 75 % ethanol, dried and dissolved in 50 µl of a TE buffer and used as a DNA sample.

### <PCR analysis>

PCR analysis was performed by using the obtained DNA sample as a template. The PCR cycle was done as follows: 35 cycles of at 96 °C for 10 sec, at 65 °C for 30 min, at 72 °C for 30 sec and further at 72 °C for 2 min. After the reaction, the PCR product was fractionated by 6 % modified polyacrylamide electrophoresis, stained by SYBR Gold and visualized using a blue light transilluminator. As a result, a band indicating polymorphism was detected at around 190 bp and 210 bp and a genotype of F1ST type was confirmed in the tested 10 varieties. It was confirmed that these bands were amplified with good reproductivity, and therefore, effective as an identification marker.

| Composition of Reaction Solution (in 25µl) | |
|---|---|
| 10X PCR buffer | 2.5 µl |
| dNTP (2.5mM) | 2 µl |
| Forward Primer* | 0.5 µM |
| Reverse Primer** | 0.5 µM |
| EX Taq (Takara) | 0.5 µl (2.5 U/µl) |
| Stevia DNA | 1 µl (30 ng/µl) |
| sterile water | fill up to 25 µl |
| * Forward Primer: CACGCGAACTCCTCGACTCGACC | |
| ** Reverse Primer: GCTGCATGCTTGCATGCATGAAATC | |

### Example 2: Preparation of a high-purity ST sweetener

### (1) Extraction

2 g of dried leaves obtained in May and September from each variety F1ST-1 to F1ST-9 were mixed to make a 18 g of mixture and extracted several times until the 20 times diluted aqueous solution loses its sweetness. The extract was run into a column loading 20 ml of an anion-exchange resin [Duolite A-4] and 5 ml of activated charcoal particles, and then, the eluate was run into a column loading 100 ml of an adsorption resin [Diaion HP-20] so that the sweetening components were absorbed. After sufficient washing with water, the column was eluted with 300 ml of methanol. The eluate was concentrated under vacuum and dried to give a pale yellowish-white powder.

### (2) Recrystallization

10 g of each of the above mentioned purified extracts (the F1ST varieties in May and September) was heated and dissolve in 10 times 95 % methanol, and then cooled at 4 °C for 6 days. The resultant crystal was separated, washed with cold methanol and dried under vacuum to give each 7.5 g and 7.4 g of white crystal.
For comparison, the SN variety was subjected to the same treatments as above and the resultant 4.6 g of white crystal (SN-CRY) was analyzed by high-performance liquid chromatography using the same conditions as those in Example 1.

The analysis results of the purified extracts are shown in Table 3. In the Table, ST represents stevioside and RA represents rebaudioside A.

**[Table 3]**

| Variety Name | ST (%) | RA (%) | Ratio of RA* | % Yield (g) |
|---|---|---|---|---|
| F1ST May | 93.8 | 0 | 0 | 75% (7.5g) |
| F1ST September | 98.4 | 0.1 | 0 | 74% (7.4g) |
| SN-CRY | 90.1 | 5.6 | 6 | 46% (4.6g) |

| | | | | |
|---|---|---|---|---|
| * Parts by weight of RA against 100 parts by weight of ST | | | | |

High-purity ST sweetener which does not substantially contain RA, can be obtained by only one recrystallization of the ST from the present invention varieties F1ST-1 to F1ST-9.

### Organoleptic Test 1

A 0.02 % solution of both F1ST May and SN-CRY powders obtained in Example 2 was prepared and bitterness, astringency and quality of sweetness thereof were compared by 10 panelists who were specialized in the quality of stevia sweeteners.

**[Table 4]**

| | | |
|---|---|---|
| Bitterness | Number of people who felt bitterness in ST variety | Number of people who felt bitterness in SN variety |
| | 4 | 6 |
| Astringency | Number of people who felt astringency in ST variety | Number of people who felt astringency in SN variety |
| | 4 | 6 |
| Quality of Sweetness | Number of people who felt clearness in quality of sweetness in ST variety | Number of people who felt clearness in quality of sweetness in SN variety |
| | 7 | 3 |

Although there are no great differences in bitterness and astringency among the samples, the F1ST variety has improved clearness in quality of taste.

### Example 3: Preparation of α-glucosylstevioside sweetener

4 g of both the powder of FIST-September obtained according to Example 2 and the powder obtained in Example 3 as well as 8 g of dextrin having DE (starch degradation rate):10 as α-glucosyl saccharide were heated to dissolve in 120 ml of water, followed by cooling to 70 °C. Calcium chloride was added to make a 1 mmol solution based on the total amount of the substrate and the pH was adjusted to 6.0. 100 units of cyclodextrin glucosyltransferase were added and reacted for 24 hours at 70 °C. Then, each reaction solution was heated at 95 °C for 30 minutes to deactivate the enzyme.

After each reaction solution was filtered to remove the suspended solids, each filtrate was run into a column loading 100 ml of synthetic adsorption resin Diaion HP-20. After sufficiently washing with water, the column was eluted with 300 ml of methanol. The eluate was run into a column loading an anion-exchange resin (amberlite IRA-94), desalted and decolorized, followed by concentration under vacuum and dried to give 2.6 g of enzyme-treated α-F1ST and 2.5 g of enzyme-treated α-F1ST-CRY α-glucosylstevioside, white powders.

### Example 4: Adjustment of added sugar chain

1 % of commercially available glucoamylase [Glucozyme, NAGASE & CO., LTD] against a solid content was added to 1.5 g of the enzyme-treated α-F1ST and the enzyme-treated α-F1ST-CRY from Example 3 and reacted for 5 hours at 50 °C. After the reaction, the enzyme was deactivated by heating at 95 °C for 30 minutes. After each reaction the solution was filtered to remove the suspended solids, each filtrate was run into a column loading 100 ml of synthetic adsorption resin Diaion HP-20. After sufficiently washing with water, the column was eluted with 300 ml of methanol. The eluate was run into a column loading an anion-exchange resin (amberlite IRA-94), desalted and decolorized, and then concentrated under vacuum and dried to give 0.7 g of added sugar chain-adjusted α-F1ST and 0.6 g of added sugar chain-adjusted α-F1ST-CRY white powders.
As a comparative study, a pale yellowish-white powder from the F1SN variety obtained in Example 2 was subjected to the same treatments as above to give enzyme-treated α-SN and added sugar chain-adjusted α-SN-CRY.

### Organoleptic Test 2

Similarly to Organoleptic Test 1, quality of sweetness was compared between the enzyme-treated α-F1ST and the enzyme-treated α-SN.

**[Table 5]**

| | | |
|---|---|---|
| Off-taste | Number of people who felt off-taste in α-F1ST | Number of people who felt off-taste in α-SN |
| | 4 | 6 |
| Aftertaste | Number of people who felt strong aftertaste in α-F1ST | Number of people who felt strong aftertaste in SN |
| | 2 | 8 |
| Quality of Sweetness | Number of people who felt good taste in α-F1ST | Number of people who felt good taste in α-SN |
| | 7 | 3 |

The aftertaste and the quality of sweetness are improved by α-glucosylation because the ST obtained from the novel stevia variety of the present invention has high purity.

### Organoleptic Test 3

Similarly to Organoleptic Test 1, quality of sweetness was compared between the added sugar chain-adjusted α-ST-CRY and the added sugar chain-adjusted α-SN-CRY.

**[Table 6]**

| | | |
|---|---|---|
| Off-taste | Number of people who felt off-taste in α-F1ST-CRY | Number of people who felt off-taste in α-SN-CRY |
| | 2 | 8 |
| Aftertaste | Number of people who felt strong aftertaste in α-F1ST-CRY | Number of people who felt strong aftertaste in α-SN-CRY |
| | 2 | 8 |
| Quality of Sweetness | Number of people who felt good taste in α-F1ST-CRY | Number of people who felt good taste in α-SN-CRY |
| | 9 | 1 |

The ST sweetener has no off-taste and improved aftertaste and sweetness quality, provided by α-glucosylation and sugar chain adjustment, as the ST obtained from the novel stevia variety of the present invention has high purity.

### INDUSTRIAL APPLICABILITY

The novel variety of Stevia Rebaudiana Bertoni of the present invention has low RA content and high ST content. Accordingly, various favorable stevioside sweeteners can be prepared from the dried leaves obtained by cultivation of the novel variety.

## Claims

1. A method for preparing a sweetener comprising extracting a plant of a Stevia Rebaudiana Bertoni variety which contains not more than 8 parts by weight of rebaudioside A against 100 parts by weight of stevioside, wherein the plant has a characteristic base sequence located at 210 bp detectable by DNA analysis using a RAPD method, or the dried leaves thereof, with water or aqueous solvent.

2. A method of claim 1 wherein the plant is obtained from the seeds of Stevia Rebaudiana Bertoni variety (Deposition No. FERM BP-10870).

3. A method for obtaining a high-purity stevioside from the sweetener obtained by the method of claim 1, wherein the stevioside contains not more than 5 parts by weight of rebaudioside against 100 parts by weight of stevioside and has a purity of more than 93 %.

4. A method for obtaining a high-purity stevioside from the sweetener obtained by the method of claim 2, wherein the stevioside contains not more than 5 parts by weight of rebaudioside against 100 parts by weight of stevioside and has a purity of more than 93 %.

5. A method for preparing α-glucosylstevioside from the sweetener obtained by the method of claim 1.

6. A method for preparing α-glucosylstevioside from the sweetener obtained by the method of claim 2.

7. A method for preparing added sugar chain-adjusted α-glucosylstevioside from the sweetener obtained by the method of claim 5.

8. A method for preparing added sugar chain-adjusted α-glucosylstevioside from the sweetener obtained by the method of claim 6.

9. A plant of a Stevia Rebaudiana Bertoni variety which contains not more than 8 parts by weight of rebaudioside A against 100 parts by weight of stevioside, wherein the plant has a characteristic base sequence located at 210 bp detectable by DNA analysis using a RAPD method.

10. A plant of claim 9, wherein the plant is obtained from the seeds of Stevia Rebaudiana Bertoni variety (Deposition No. FERM BP-10870).

11. A method for selecting the Stevia Rebaudiana Bertoni variety of claim 9 by DNA analysis using a RAPD method wherein the following sequences are used as a primer:
Forward primer: CACGCGAACTCCTCGACTCGACC
Reverse primer: GCTGCATGCTTGCATGCATGAAATC
